# EUROPÄISCHE PATENTSCHRIFT

(11) **EP 3 422 996 B1**
(45) Veröffentlichungstag und Bekanntmachung des Hinweises auf die Patenterteilung: **25.08.2021**
(21) Anmeldenummer: 17707895.3
(22) Anmeldetag: 01.03.2017
(51) Int. Cl.: A61C 13/15, A61C 9/00, A61C 19/045, A61B 5/11, A61B 5/00, A61C 11/00, A61C 11/06

(54) **VORRICHTUNG UND VERFAHREN ZUR VERMESSUNG EINER UNTERKIEFERBEWEGUNG**
DEVICE AND METHOD FOR MEASURING A MOVEMENT OF A MANDIBLE
DISPOSITIF ET PROCÉDÉ POUR MESURER UN MOUVEMENT DU MAXILLAIRE INFÉRIEUR

(30) Priorität: 01.03.2016 DE 102016103655; 13.01.2017 DE 102017200515
(43) Veröffentlichungstag der Anmeldung: 09.01.2019
(73) Patentinhaber: Ignident GmbH, 67059 Ludwigshafen am Rhein (DE)
(72) Erfinder: CLAUSS, Petra, Ina, 83700 Rottach-Egern (DE)
(74) Vertreter: Fish & Richardson P.C.
(86) Internationale Anmeldenummer: PCT/EP2017/054762
(87) Internationale Veröffentlichungsnummer: WO 2017/149010

(56) Entgegenhaltungen:
- DE-A1- 10 218 435
- DE-T5-112005 000 700
- DE-T5-112005 000 700
- JP-B1- 2 989 600
- JP-B1- 2 989 600
- US-A1- 2011 053 110

## Beschreibung

Die Erfindung betrifft eine Vorrichtung zur Vermessung einer Relativposition und/oder -bewegung von einem Unterkiefer relativ zu einem Oberkiefer eines Patienten. Die Vorrichtung weist eine Spule zum Erzeugen eines magnetischen Messfeldes sowie Sensoren auf, welche mit dem Ober- und Unterkiefer verbunden werden.

Ferner weist die Vorrichtung eine Auswerteeinrichtung auf, welche auf Basis der Sensorsignale die Relativposition und/oder -bewegung des Unterkiefers relativ zu dem Oberkiefer bestimmt. Die Erfindung betrifft auch ein entsprechendes Verfahren mit dieser Vorrichtung.

In der Dentaltechnik werden zu verschiedensten Zwecken digitale oder körperliche Abformungen vom Unterkiefer und vom Oberkiefer eines Patienten benötigt. Eine übliche Art der körperlichen Abformung ist beispielsweise die Verwendung einer Abformeinrichtung, insbesondere eines Abformlöffels, wobei eine Abformung der Oberkieferzähne samt Weichgewebe beziehungsweise der Unterkieferzähne samt Weichgewebe in eine Abformmasse eingedrückt wird. Es ist auch möglich, mittels Intraoralscanner und digitaler Bildverarbeitung digitale Abdrücke der Zähne des Oberkiefers und des Unterkiefers zu erfassen.

Beide Arten der Erzeugung der Abformungen von Oberkiefer beziehungsweise Unterkiefer haben jedoch die Einschränkung, dass weder eine Artikulation, insbesondere der Schlussbiss, noch die Bewegung des Unterkiefers zum Oberkiefer abgebildet werden können. Allerdings ist es zum Beispiel bei der Anfertigung von Zahnersatz notwendig, auch diese Bewegung des Unterkiefers zum Oberkiefer zu berücksichtigen. Aus diesem Grund werden oftmals die Beziehungen zwischen Unterkiefer, Oberkiefer und dem Kiefergelenk über Hilfsmittel, wie zum Beispiel Gesichtsbögen etc., ausgemessen und auf digitale (virtuelle) oder reale Artikulatoren übertragen. Diese Verfahren sind jedoch aufgrund der indirekten Vermessung der Bewegung vergleichsweise ungenau. Hier kann die Bewegung somit nur nach Annahmen simuliert werden.

In der Druckschrift DE 102 18 435 A1 wird ein Verfahren und eine Vorrichtung zur dreidimensionalen Bewegungsanalyse von Zahnoberflächen des Oberkiefers in Relation zum Unterkiefer vorgestellt. Die Vorrichtung weist einen Oberkiefersensor auf, welcher an einem Gesichtsbogen angeordnet ist und die Bewegung einer Bissplatte, welche fest mit dem Oberkiefer verbunden ist, aufnimmt. Ferner weist die Vorrichtung einen Unterkiefersensor auf, welcher über einen Behelf mechanisch starr mit dem Unterkiefer verbunden ist. Durch die Auswertung der Signale des Oberkiefersensors und des Unterkiefersensors kann über eine Referenzierung der Sensoren relativ zu dem Oberkiefer und zu dem Unterkiefer eine Position und damit auch eine Bewegung des Unterkiefers relativ zu dem Oberkiefer aufgezeichnet werden.

Die Druckschrift DE 11 2005 000 700 T5 offenbart eine Vorrichtung zur Messung einer Position eines Unterkiefers relativ zu einem Oberkiefer, wobei Magnetfeldsensoren und Magnetfeldgeneratoren in dem Mund des Patienten angeordnet werden, um die Positionen zu bestimmen.

Eine Vorrichtung gemäß dem Oberbegriff des Anspruchs 1 ist aus der US 2011/053110 A1 bekannt.

Der Erfindung liegt die Aufgabe zugrunde, eine Vorrichtung und ein Verfahren zur Vermessung einer Relativposition und/oder -bewegung von einem Unterkiefer relativ zu einem Oberkiefer eines Patienten vorzuschlagen, welche sich durch eine hohe Messgenauigkeit auszeichnen.

Diese Aufgabe wird durch eine Vorrichtung mit den Merkmalen des Anspruchs 1 sowie einem Verfahren mit den Merkmalen des Anspruchs 6 gelöst. Bevorzugte oder vorteilhafte Ausführungsformen der Erfindung ergeben sich aus den Unteransprüchen, der nachfolgenden Beschreibung sowie den beigefügten Figuren.

Gegenstand der Erfindung ist eine Vorrichtung, welche zur Vermessung einer Relativposition und/oder -bewegung von einem Unterkiefer relativ zu einem Oberkiefer eines Patienten geeignet und/oder ausgebildet ist. Die Vorrichtung dient dazu, die Relativposition des Unterkiefers relativ zu dem Oberkiefer des Patienten zu bestimmen. Werden mehrere derartige Relativpositionen in einer zeitlichen Folge aufgenommen, so kann die Vorrichtung auch dazu dienen, eine Relativbewegung von dem Unterkiefer relativ zu dem Oberkiefer des Patienten zu bestimmen.

Die Vorrichtung basiert auf einem elektromagnetischen Messprinzip. So umfasst die Vorrichtung mindestens oder genau eine Spule, im Nachfolgenden auch als "Senderspule" bezeichnet, welche zum Erzeugen eines magnetischen Messfelds ausgebildet ist. Vorzugsweise ist die Senderspule so platziert, dass das magnetische Messfeld mit einem Kopf des Patienten, insbesondere dem Bereich des Unterkiefers und des Oberkiefers, überlagert und/oder überlagerbar ist.

Die Vorrichtung umfasst mindestens oder genau einen Unterkiefersensor, welcher intraoral, das heißt im Mundraum, insbesondere vollständig im Mundraum, angeordnet ist.

Ferner weist die Vorrichtung bevorzugt mindestens oder genau einen Oberkiefersensor auf, wobei der Oberkiefersensor ebenfalls intraoral, das heißt im Mundraum, insbesondere vollständig im Mundraum, des Patienten angeordnet ist.

Messtechnisch ist der Unterkiefer der wichtige Kiefer, da dieser sich bewegt. Der Unterkiefersensor nimmt somit die Relativbewegung auf. Der Oberkiefersensor bildet vorzugsweise einen Referenzsensor zum Unterkiefersensor. Der Oberkiefersensor kann darüber hinaus eine Kopfbewegung über ein Computerprogramm rechnerisch eliminieren.

Der Unterkiefersensor und der Oberkiefersensor - nachfolgend zusammenfassend auch als Sensoren bezeichnet - sind als Positionssensoren jeweils zur Bestimmung von einer Position, insbesondere einer absoluten Position, in dem Messfeld und/oder relativ zu der Senderspule ausgebildet. Insbesondere sind die Sensoren ausgebildet, mindestens drei translatorische Freiheitsgrade zu bestimmen.

Derartige Positionssensoren, welche in einem magnetischen Messfeld, insbesondere Wechselmessfeld, eine Position bestimmen können, sind beispielsweise aus der Druckschrift WO 97/36192 A1 oder aus der Druckschrift EP 1 303 771 B1 bekannt, deren Offenbarungsgehalt via Referenzierung in die vorliegende Anmeldung übernommen wird.

Ferner umfasst die Vorrichtung eine Auswerteeinrichtung, welche besonders bevorzugt als eine digitale Datenverarbeitungseinrichtung, wie zum Beispiel ein Computer, Mikrocontroller, etc. ausgebildet ist, welche zur Bestimmung der Relativposition und/oder Relativbewegung des Unterkiefers relativ zu dem Oberkiefer auf Basis der von den Sensoren bestimmten Positionen ausgebildet ist. Nachdem die Sensoren fest mit dem Unterkiefer beziehungsweise Oberkiefer verbunden sind und die Positionen über die Auswerteeinrichtung erfasst werden können, ist die Lage, insbesondere die Relativlage und/oder Relativbewegung zwischen Oberkiefer und Unterkiefer aus den vorliegenden Daten einfach bestimmbar. Vorzugsweise wird über eine zusätzliche Sensoreinrichtung (Probe) die Position der Sensoren bestimmt, so dass diese in der Auswerteeinrichtung referenzierbar sind.

Es ist dabei ein Vorteil, dass die Sensoren intraoral angeordnet sind und dadurch keine Verlängerungen oder andere Apparaturen gebraucht werden, welche das Messergebnis verfälschen könnten. Vielmehr werden die Positionen unmittelbar dort aufgenommen, wo diese am genauesten sind, nämlich an den Zähnen des Unterkiefers beziehungsweise des Oberkiefers. Die Messtechnik über die insbesondere als Magnetfeldsensoren ausgebildeten Sensoren ist mittlerweile etabliert, sodass hier auch keine großen Messungenauigkeiten zu erwarten sind.

Somit stellt die Vorrichtung ein Messsystem dar, über welches die Relativposition und/oder Relativbewegung von Unterkiefer zu Oberkiefer mit einer hohen Genauigkeit und einem geringen konstruktiven Aufwand sehr genau aufgenommen werden kann.

Im Rahmen der Erfindung wird vorgeschlagen, dass die Senderspule extraoral, insbesondere benachbart zu dem Kopf oder oberhalb des Kopfes des Patienten angeordnet ist. Somit durchgreift das Messfeld den Kopf des Patienten. Besonders bevorzugt ist die Senderspule stationär und/oder unabhängig von dem Patienten angeordnet. Diese stationäre Anordnung der Senderspule unabhängig von dem Patienten hat den Vorteil, dass die Senderspule bei der Messung sich nicht bewegen kann und damit eine sehr genaue Referenz bildet.

Der Erfindung liegt die Überlegung zugrunde, dass das Magnetfeld homogener ausgeprägt ist, wenn dieses durch eine Senderspule erzeugt wird, welche sich nicht in den Mundraum des Patienten integrieren muss, sondern nahezu ohne jegliche Bauraumeinschränkung außerhalb des Mundraums angeordnet werden kann. Auch ist es ohne die Bauraumbeschränkung möglich, die Leistung für das Messfeld frei oder nahezu frei zu wählen. Beide Vorteile führen letztlich zu einem stabileren Messfeld und damit zu genaueren Messergebnissen. Die Senderspule kann das Messfeld über Wechselstrom oder über einen pulsierenden Gleichstrom generieren.

Bei einer bevorzugten Ausgestaltung der Erfindung ist die Senderspule seitlich schräg oder oberhalb zu dem Patienten oder zu dem Messraum, in dem der Patient positioniert werden soll, angeordnet. Insbesondere durchstrahlt das Messfeld ausgehend von der Senderspule eine Backe des Patienten. Diese Positionierung hat den Vorteil, dass nur Gewebeteile des Patienten von dem Messfeld durchdrungen werden müssen.

Bei einer bevorzugten Ausgestaltung der Erfindung sind die Sensoren ausgebildet, zudem mindestens zwei Rotationsfreiheitsgrade, vorzugsweise alle drei Rotationsfreiheitsgrade bzw. sechs Freiheitsgrade (drei Translationsfreiheitsgrade und drei Rotationsfreiheitsgrade), in dem Messfeld zu bestimmen. Die Sensoren sind insbesondere als fünf DOF-Sensoren (degrees of freedom) oder sogar als sechs DOF-Sensoren ausgebildet. In der Ausbildung als fünf DOF-Sensoren können alle drei translatorischen Freiheitsgrade und zwei rotatorische Freiheitsgrade erfasst werden. In der Ausbildung als sechs DOF-Sensoren können alle drei translatorischen Freiheitsgrade und alle drei rotatorischen Freiheitsgrade erfasst werden. Durch die Erfassung der Rotationsfreiheitsgrade erfolgt wiederum eine verbesserte Messung, sodass die Messgenauigkeit der Vorrichtung weiter gesteigert werden kann. In einem Ausführungsbeispiel bestehen die Sensoren aus 2 5DOF Sensoren, die dadurch zu einem 6 DOF Sensor werden. Damit können zwei Messpunkte im Unterkiefer und zwei Messpunkte im Oberkiefer, d.h. vier Messpunkte in x-, y-, und z-Richtung aufgezeichnet werden, um daraus eine Relativbewegung zu bestimmen.

In einer besonders bevorzugten Konfiguration der Erfindung sind an dem Oberkiefer und an dem Unterkiefer jeweils wahlweise genau zwei fünf DOF-Sensoren oder genau ein sechs DOF-Sensor angeordnet. Insbesondere ist an dem Oberkiefer genau ein sechs DOF-Sensor und an dem Unterkiefer genau ein sechs DOF-Sensor angeordnet.

Bei einer bevorzugten Ausgestaltung der Erfindung ist an dem Unterkiefer und an dem Oberkiefer jeweils ein Sensor, insbesondere jeweils ein sechs DOF-Sensor, im Prämolarenbereich, insbesondere diametral gegenüberliegend angeordnet. Wenn der Unterkiefersensor rechts angeordnet ist, dann ist der Oberkiefersensor links positioniert oder wenn der Unterkiefersensor links angeordnet ist, dann ist der Oberkiefersensor rechts positioniert. Durch diese Anordnung insbesondere in Verbindung mit der seitlich angeordneten Senderspule wird die Messgenauigkeit weiter verbessert.

Gemäß der Erfindung ist der kleinste Abstand zwischen dem jeweiligen Sensor und dem Zahn, auf dem der Sensor angeordnet ist, kleiner als 0,5 Zentimeter und insbesondere kleiner als 0,3 Zentimeter ausgebildet. Durch die nahe Positionierung der Sensoren an den Zähnen wird erreicht, dass Messfehler aufgrund von Beabstandungen oder Verlängerungen zwischen den Sensoren und den Zähnen vermieden werden.

Gemäß der Erfindung weist die Vorrichtung eine Halteeinrichtung je Kiefer auf, wobei die Halteeinrichtungen an dem Unterkiefer und/oder an dem Oberkiefer befestigt sind und die jeweiligen Sensoren aufnehmen. Die Halteeinrichtungen bilden somit eine mechanische Verbindung zwischen den Zähnen und den Sensoren. Erfindungsgemäß sind die jeweiligen Halteeinrichtungen an dem Oberkiefer (OK) und/oder dem Unterkiefer (UK) als Sensorschuh ausgebildet, der auf die jeweiligen Zähne aufklebbar ist.

Die erfindungsgemäße Halteeinrichtung für mindestens einen Oberkiefersensor (OS1) und/oder mindestens einen Unterkiefersensor (US1), insbesondere zur Verwendung in der genannten Vorrichtung zur Vermessung einer Relativposition und/oder -bewegung von einem Unterkiefer (UK) relativ zu einem Oberkiefer (OK) eines Patienten, ist an dem Oberkiefer (OK) und/oder an dem Unterkiefer (UK) befestigbar.

Der Sensorschuh weist zumindest einen gekrümmten Oberflächenbereich und/oder zumindest eine Positionsmarkierung auf.

Vorzugsweise ist die Positionsmarkierung als Einbuchtung oder Ausnehmung in dem vom jeweiligen Zahn abgewandten gekrümmten Oberflächenbereich des Sensorschuhs ausgebildet.

Weiter bevorzugt ist die Positionsmarkierung als kegelförmige Ausnehmung ausgebildet, deren Spitze einen Nullpunkt der Relation des Sensors zur Relativposition und/oder -bewegung vom Unterkiefer (UK) relativ zum Oberkiefer (OK) definiert.

Dabei steht die Spitze der Positionsmarkierung in direkter Relation zu einem planaren Oberflächenbereich um die Ausnehmung des Sensorschuhs, wobei die Sensorposition in Bezug auf die Relativposition und/oder -bewegung vom Unterkiefer (UK) relativ zum Oberkiefer (OK) definiert ist.

Bevorzugt ist die Positionsmarkierung auf dem Sensorschuh mittig angeordnet.

Der Sensorschuh weist zumindest einen dem jeweiligen Zahn oder Zähnen zugewandten gekrümmten Oberflächenbereich auf und ist zur formschlüssigen Aufnahme eines Oberkiefersensors (OS1) und/oder eines Unterkiefersensors (US1) ausgebildet.

Bei einer besonders bevorzugten Ausgestaltung der Erfindung umfasst die Vorrichtung eine Digitalisierungseinrichtung zur Erstellung eines digitalen, dreidimensionalen Modells des Oberkiefers und/oder des Unterkiefers, wobei in dem Modell die Halteeinrichtungen und/oder die Sensoren modelliert sind. Beispielsweise kann die Digitalisierungseinrichtung als ein Intraoralscanner ausgebildet sein. Bei anderen Ausführungsformen der Erfindung können der Oberkiefer und der Unterkiefer des Patienten auch mechanisch oder körperlich abgeformt werden und nachfolgend in einem 3D-Scanner digitalisiert werden. Bei beiden Ausführungsformen ist jedoch vorgesehen, dass die Halteeinrichtung und/oder die Sensoren mit abgeformt beziehungsweise digitalisiert werden, sodass die Relativlage zwischen den Sensoren beziehungsweise Halteeinrichtungen und dem Oberkiefer und dem Unterkiefer eindeutig bestimmt sind. Alternativ hierzu wird eine weitere Sensoreinrichtung (Probe) verwendet. Damit kann die Position der Sensoren im Mund bestimmt und in die Digitalisierungseinrichtung übertragen werden. Die Sensoreinrichtung ist insbesondere eine Sensorspitze mit der an mind. 3 wiederauffindbaren Punkten jeweils ein Orientierungspunkt aufgezeichnet wird. Diese Punkte referenzieren dann zu den Sensoren. Eine entsprechende Software berechnet die Position der aufgeklebten Sensoren.

Auf diese Weise ist es dann bei der nachfolgenden Datenverarbeitung möglich, nicht nur die Relativposition und Relativbewegung der Sensoren zueinander als Bewegung des Unterkiefers relativ zu dem Oberkiefer zu bestimmen, sondern die Kontur der Zähne des Oberkiefers und die Kontur der Zähne des Unterkiefers relativ zueinander zu modellieren.

Ein weiterer Gegenstand der Erfindung betrifft ein Verfahren zur Bestimmung der Relativposition und/oder der Relativbewegung von einem Unterkiefer relativ zu einem Oberkiefer des Patienten, wobei eine Vorrichtung eingesetzt wird, wie diese zuvor beschrieben wurde.

Bei dem Verfahren ist vorgesehen, dass die Sensoren an dem Unterkiefer und an dem Oberkiefer angeordnet werden und Sensorsignale der Sensoren aufgenommen werden. In einem nachfolgenden Schritt erfolgt eine Bestimmung der Relativposition und/oder -bewegung zwischen dem Oberkiefer und dem Unterkiefer auf Basis der Sensorsignale.

Optional umfasst das Verfahren einen Schritt zur Erstellung eines digitalen, dreidimensionalen Modells des Oberkiefers und/oder des Unterkiefers, wobei in dem Modell die Halteeinrichtung und/oder die Sensoren modelliert sind. In einem weiteren Schritt werden insbesondere in der Auswerteeinrichtung die Daten der Sensoren mit dem Modell des Oberkiefers und des Unterkiefers fusioniert, so dass ein Modell des Oberkiefers und des Unterkiefers in verschiedenen Relativpositionen und/oder in Relativbewegung zueinander gebildet ist. Insbesondere umfasst das Modell einen Bewegungsablauf, wobei der Bewegungsablauf mehrere komplexe Einzelbewegungsabläufe wie Öffnen, Schließen, Kauen etc. umfasst. Auf diese Weise können Bewegungsbahnen des Unterkiefers relativ zu dem Oberkiefer bestimmt werden.

Eine erfindungsgemäße Vorrichtung zur Simulation und Übertragung einer gemessenen Relativbewegung von einem Unterkiefer (UK) relativ zu einem Oberkiefer (OK) eines Patienten aus dem genannten Verfahren, umfasst einen Empfänger zum Empfangen eines digitalen, dreidimensionalen Modells des Oberkiefers (OK) und/oder des Unterkiefers (UK), wobei in dem Modell die Halteeinrichtungen bzw. Sensorschuhe und/oder Sensoren zum Empfang von Daten positioniert sind,

Weitere Merkmale, Vorteile und Wirkungen der Erfindung ergeben sich aus der nachfolgenden Beschreibung eines bevorzugten Ausführungsbeispiels der Erfindung sowie der beigefügten Figuren. Dabei zeigen:
Figur 1 a, b eine schematische Draufsicht auf einen Oberkiefer beziehungsweise Unterkiefer;
Figur 2 ein Blockdiagramm als ein Ausführungsbeispiel einer erfindungsgemäßen Vorrichtung zur Messung einer Relativposition und/oder -bewegung von einem Unterkiefer relativ zu einem Oberkiefer eines Patienten,
Figur 3 eine schematische Darstellung eines Messaufbaus;
Figuren 4-8 verschiedene Schrägansichten einer U-förmig ausgebildeten Bissplatte für einen Oberkiefer oder Unterkiefer, wobei die Bissplatte nicht Teil der erfindungsgemäßen Vorrichtung ist;
Figuren 9-11 eine schematische Seitenansicht, Draufsicht und Schrägansicht eines erfindungsgemäßen Sensorschuhs;
Figur 12 einen Artikulator; und
Figuren 13 und 14 einen Übertragungstisch mit Bewegungssimulator.

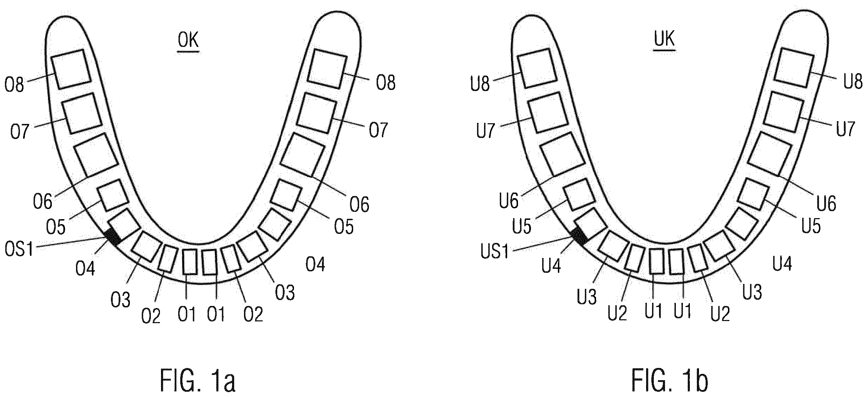

Die Figur 1a und 1b zeigen in einer stark schematisierten Darstellung einen Oberkiefer OK (Fig. 1a) beziehungsweise Unterkiefer UK (Fig. 1b) in einer Draufsicht. Bei jedem der Kiefer sind sechzehn Zähne eingezeichnet, wobei die Zähne des Oberkiefers OK ausgehend von den Schneidezähnen mit O1 und O2 über die Prämolaren O4 und O5, über die Molaren bis hin zu den Weisheitszähnen mit O8 durchgehend nummeriert sind. In gleicher Weise sind die Zahne des Unterkiefers UK ausgehend von den Schneidezähnen mit U1 über die Molaren bis zu den Weisheitszähnen U8 durchnummeriert.

An dem Oberkiefer OK ist ein Sensor OS1 angeordnet. Optional können noch weitere Sensoren vorgesehen sein. Der Oberkiefersensor OS1 ist an einem oder beiden Prämolaren O4 und/oder O5 befestigt. In ähnlicher Weise ist an dem Unterkiefer UK ein Unterkiefersensor US1 angeordnet. Der Unterkiefersensor US 1 ist an dem oder den Prämolaren U4 und/oder U5 angeordnet. Optional können weitere Sensoren an dem Oberkiefer und/oder an dem Unterkiefer vorgesehen sein.

Wie sich der Draufsicht entnehmen lasst, sind die Sensoren OS1 beziehungsweise US1 unmittelbar an den jeweiligen Zähnen O4 beziehungsweise U4 angeordnet. Der Abstand zwischen den Sensoren und den Zähnen beträgt jeweils weniger als 0,5 Zentimeter. Insbesondere sind die Sensoren unmittelbar benachbart zu den Zähnen positioniert. Alle Sensoren OS1 sowie US1 sind intraoral, das heißt in der Mundhöhle des Patienten, angeordnet. Die Sensoren sind mit den jeweiligen Zähnen fest verbunden, sodass diese jeweils eine örtliche Referenz zu den Zähnen und damit zu dem Oberkiefer OK beziehungsweise Unterkiefer UK bilden.

Die Sensoren sind auf gegenüberliegenden Hälften der Kiefer UK, OK angeordnet, sodass einer der Sensor auf der linken Seite des Kiefers UK, OK und der andere Sensor auf der rechten Seite des Kiefers OK, UK angeordnet ist.

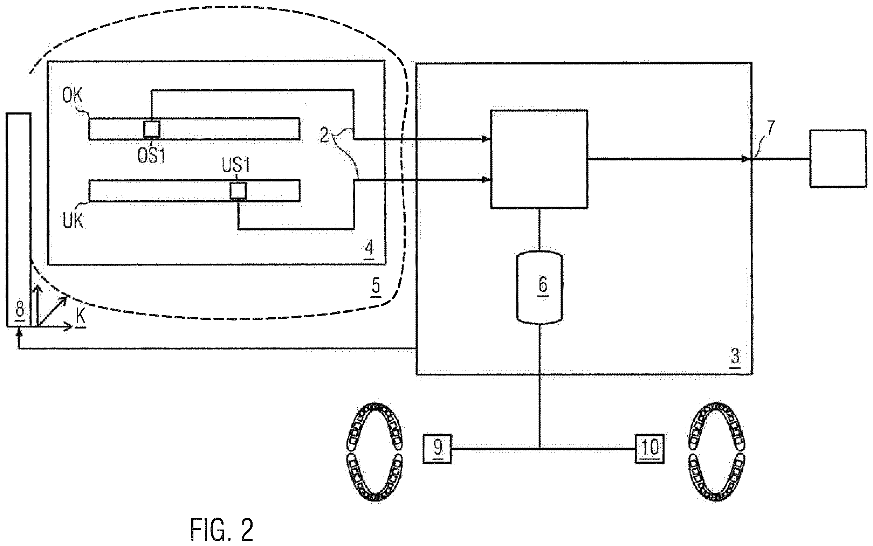

In der Figur 2 ist ein schematisches Blockdiagramm einer Vorrichtung 1 zur Vermessung der Relativposition und/oder -bewegung von dem Unterkiefer UK relativ zu dem Oberkiefer OK dargestellt. Die Relativposition und/oder Relativbewegung wird an einem Menschen vermessen, sodass dessen natürliche Kieferbewegung zum Beispiel beim Kauen, Öffnen und Schließen, Seitwärtsbewegung links, Seitwärtsbewegung rechts, Protrusion und Retrusion aufgenommen wird. Auf der linken Seite ist stark schematisiert nochmals der Oberkiefer OK und der Unterkiefer UK mit den Sensoren OS1 sowie US1 gezeigt. In dieser Darstellung ist jedoch eine grob schematische Frontansicht dargestellt. Die Sensoren sind über eine Kabelverbindung 2 mit einer Auswerteeinrichtung 3 verbunden, wobei über die Kabeleinrichtung 2 Sensorsignale von den Sensoren in die Auswerteeinrichtung 3 geführt werden.

Die Vorrichtung 1 weist eine Senderspule 8 auf, welche benachbart zu einem Kopf 4 des Patienten (grob schematisiert als Rechteck dargestellt) angeordnet ist. Die Senderspule 8 erzeugt ein elektromagnetisches Messfeld 5, welches den Kopf 4 des Patienten durchdringt und van den Sensoren OS1 sowie US1 erfasst werden kann.

Die Sensoren sind als Magnetfeldsensoren ausgebildet und ermöglichen es, zumindest eine absolute Position in dem Messfeld 5 zu erfassen. Somit kann aus den Sensorsignalen eine absolute Position der Sensoren relativ zu der Senderspule 8 bestimmt werden. Die absolute Position kann beispielsweise in einem Koordinatensystem K, welches ortsfest mit der Senderspule 3 verbunden ist, als XYZ-Koordinaten ausgegeben werden. Optional können die Sensoren neben der absoluten Position, also drei translatorischen Freiheitsgraden, weitere, insbesondere rotatorische Freiheitsgrade, erfassen. Die Sensoren US1 und OS1 sind in diesem Ausführungsbeispiel jeweils als ein sechs-DOF-Sensor ausgebildet und sind somit Magnetfeldsensoren, die drei translatorische und drei rotatorische Freiheitsgrade in dem Magnetfeld als Messfeld aufnehmen können. Die Sensorsignale werden über die Kabeleinrichtung 2 an die Auswerteeinrichtung 3 weitergeleitet und dort weiterverarbeitet. Die Auswerteeinrichtung 3 ist beispielsweise als ein Computer ausgebildet oder als eine andere digitale Datenverarbeitungseinrichtung.

Die Auswerteeinrichtung 3 weist eine Speichereinrichtung 6 auf, in der ein 3D-Modell des Oberkiefers OK und des Unterkiefers UK abgelegt ist. In dem 30-Modell sind jeweils die Sensoren OS1 beziehungsweise US1 eingetragen und/oder modelliert. Nachdem über die Sensoren die Positionen relativ zu der Senderspule 8 bekannt sind, können auch die 3D-Modelle des Oberkiefers OK beziehungsweise des Unterkiefers UK lagerichtig zueinander virtuell angeordnet werden. Somit kann ein Gesamtmodell gebildet werden, in dem die 3D-Modelle des Unterkiefers UK und des Oberkiefers OK lagerichtig zueinander positioniert sind, sodass die Relativposition zueinander bestimmt ist. Ferner kann auch eine Relativbewegung von dem Oberkiefer OK und dem Unterkiefer UK in dem Gesamtmodell dargestellt werden. Das Gesamtmodell kann dann über eine Schnittstelle 7 ausgegeben werden, um zum Beispiel in einem virtuellen Artikulator und/oder in einem CAD-System weiter verwendet werden zu können. Insbesondere erlaubt die Vorrichtung einen Bewegungsablauf, wobei der Bewegungsablauf mehrere komplexe Einzelbewegungsabläufe wie Öffnen, Schließen, Kauen etc. umfasst, auszugeben und z.B. wie einen Film darzustellen. Auf diese Weise können Bewegungsbahnen des Unterkiefers relativ zu dem Oberkiefer bestimmt werden.

Die 3D-Modelle des Oberkiefers beziehungsweise des Unterkiefers werden zum Beispiel über einen Intraoralscanner 9, welcher den Oberkiefer OK beziehungsweise den Unterkiefer UK mit den aufgesetzten Sensoren aufnimmt, bereitgestellt. Alternativ hierzu wird ein Abdruck des Oberkiefers OK beziehungsweise Unterkiefers UK mit einem Abdruck der Sensoren über einen 3D-Scanner 10 digitalisiert, um die 3D-Modelle zu erhalten.

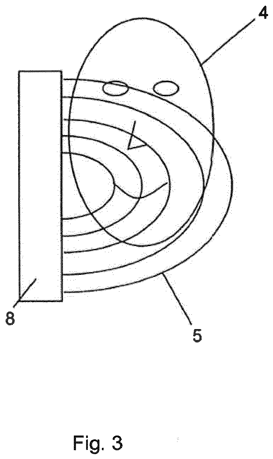

In der Figur 3 ist der Messaufbau stark schematisiert dargestellt, wobei zu erkennen ist, dass die Senderspule 8 außerhalb des Kopfes 4 des Patienten und zwar auf einer Seite angeordnet ist, so dass der Kopf 4 von dem Messfeld 5 von der Seite durchstrahlt wird. Die Senderspule 8 ist stationär und/oder unabhängig von dem Patienten angeordnet. Das Messvolumen beträgt 30 cm^3 bis 50 cm^3.

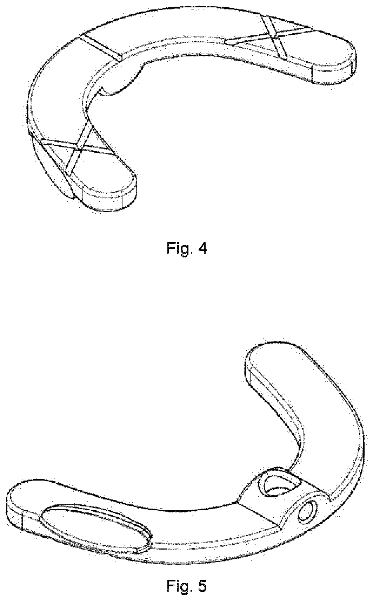

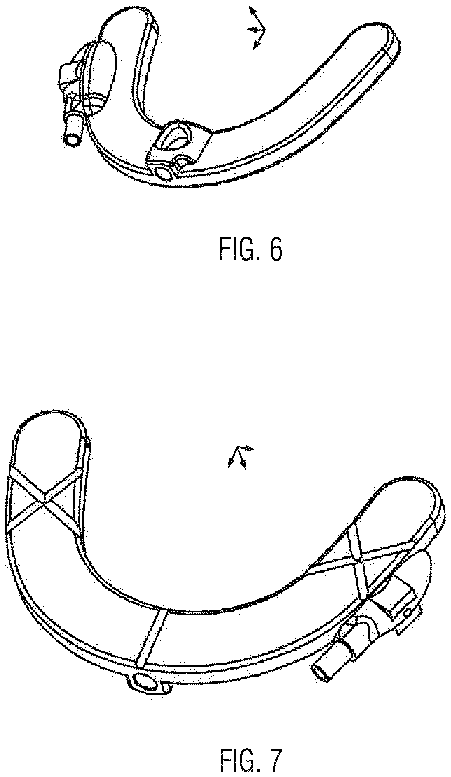

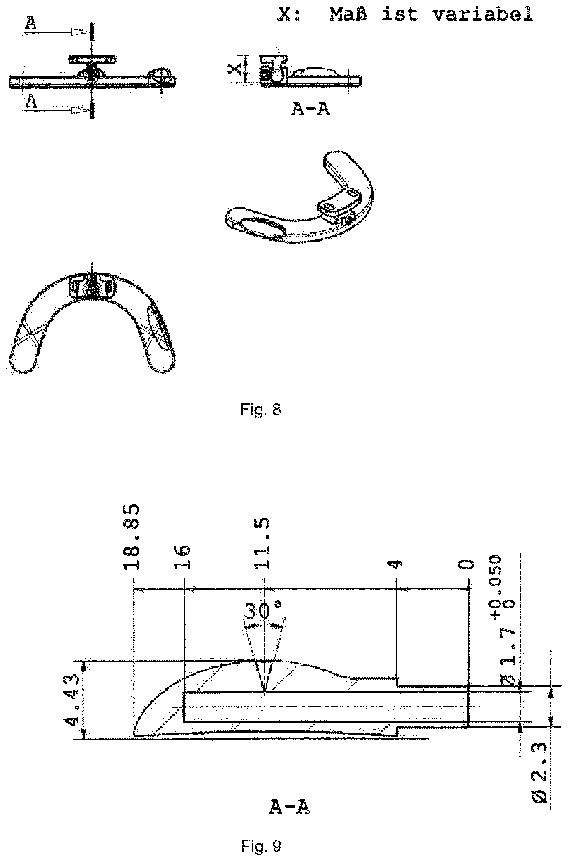

In den Figuren 4-7 sind verschiedene Schrägansichten einer U-förmig ausgebildeten Bissplatte für einen Oberkiefer oder Unterkiefer für zahnlose Patienten dargestellt. Fig. 8 zeigt zusätzlich im Detail eine Distanzierungseinrichtung mit Bissplatten, wobei die Bissplatten zwischen Unterkiefer und Oberkiefer angeordnet sind.

Die Distanzierungseinrichtung dient zur Festlegung der Kauebene und Bissnahme bei der Herstellung einer totalen Ober- und/ oder Unterkieferprothese.

Die Distanzierungseinrichtung umfasst Bissplatten, wobei eine Bissplatte am Unterkiefer angeordnet ist und eine Bissplatte am Oberkiefer angeordnet ist.

Durch Einstellen des Stiftes mit Kugel in vertikaler Richtung und Neigung kann die Distanzierungseinrichtung die richtige Lage und Neigung der Kiefer zueinander bestimmen und erleichtert das Vermessen der Kauebene für die Erstellung von Prothesen.

Alternativ kann die Distanzierungseinrichtung mehrere Stifte aufweisen, wobei die Stifte im hinteren und/oder seitlichen Bereich der Bissplatten angeordnet sind und kein Kugelgelenk aufweisen.

Besonders vorteilhaft ermöglicht es die Bissplatte, dass der Patient selbständig in eine anatomisch korrekte Kondylenposition zurückfindet, auch wenn diese vorher verloren war. Mit der Einrichtung zum Anbringen der Marker kann zudem am zahnlosen Patienten eine UK-Aufzeichnung erstellt werden.

Die jeweilige Bissplatte weist einen mittig angeordneten Stift oder mehrere Stifte auf, wobei zumindest der Stift eine Kugel mit einer Gewindebohrung aufweist, in welcher der Stift zumindest vertikal verstellbar angeordnet ist, vgl. Fig. 8. Die Bissplatten bestehen aus einem Metallfuß, Stift und Kugelkopf und einer Kunststoffplatte, die auf dem Kugelkopf mit einem Inbus verschraubt werden kann. Die Stift-Kugelbasis wird über den Metallfuß auf dem Modell in Löffelmaterial (Kunststoff) jeweils im Oberkiefer und Unterkiefer fixiert. Die beiden Bissplatten werden dabei in einer mittelwertigen vertikalen Höhe im Oberkiefer sowie Unterkiefer eingestellt und wirken im Mund des Patienten frei gegeneinander. Im Mund des Patienten wird zuerst die OK-Platte ausgerichtet (Ala-Tragus Linie, Bipupillarlinie) und über eine Schraube arretiert. Anschließend beißt der Patient zu und die UK-Platte richtet sich selbständig ohne Zutun des Zahnarztes aus. Im Anschluss kann der Zahnarzt die Bissplatten miteinander verschlüsseln.

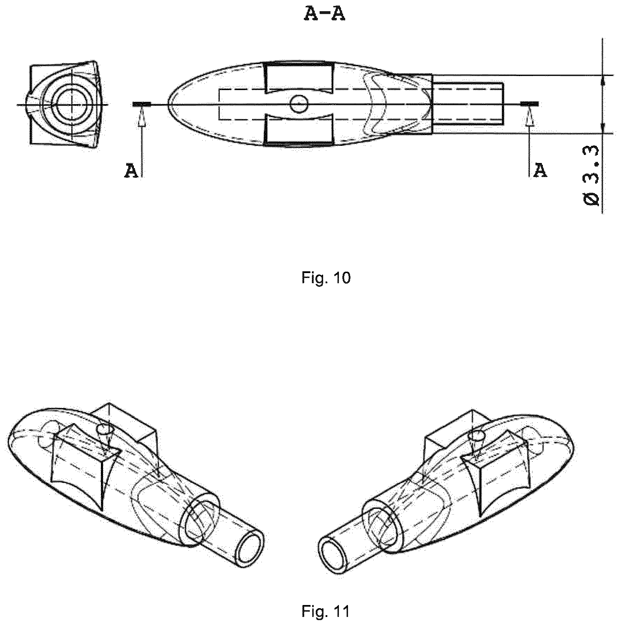

Die Figuren 9-11 zeigen eine schematische Seitenansicht, Draufsicht und Schrägansicht eines Sensorschuhs, der auf die jeweiligen Zähne oder einen Unterkieferbehelf des Unterkiefers (UK) oder einem Oberkieferbehelf des Oberkiefers (OK) befestigbar ist. Der Sensorschuh ist in seiner Form mit leicht gewölbter Sohle so gewählt, dass er leicht auf die Zähne aufzukleben ist und auch bei geringen Platzverhältnissen eingesetzt werden kann.

Eine Positionsmarkierung ist als Einbuchtung oder Ausnehmung in dem vom jeweiligen Zahn abgewandten gekrümmten Oberflächenbereich des Sensorschuhs ausgebildet. Insbesondere ist die Positionsmarkierung als kegelförmige Ausnehmung ausgebildet, so dass deren Spitze einen Nullpunkt der Relation des Sensors zur Relativposition und/oder -bewegung vom Unterkiefer (UK) relativ zum Oberkiefer (OK) definiert. Die Spitze der Positionsmarkierung steht in direkter Relation zu einem planaren Oberflächenbereich um die Ausnehmung des Sensorschuhs, wobei die Sensorposition in Bezug auf die Relativposition und/oder-bewegung vom Unterkiefer (UK) relativ zum Oberkiefer (OK) definiert ist.

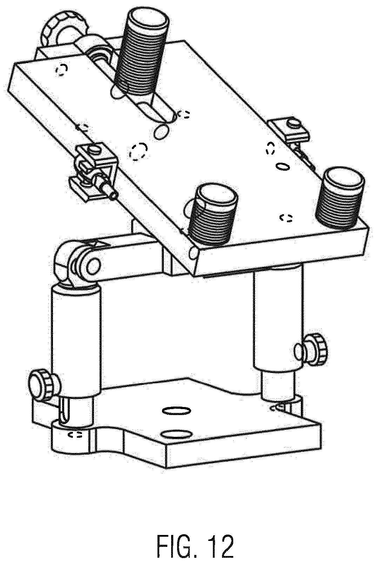

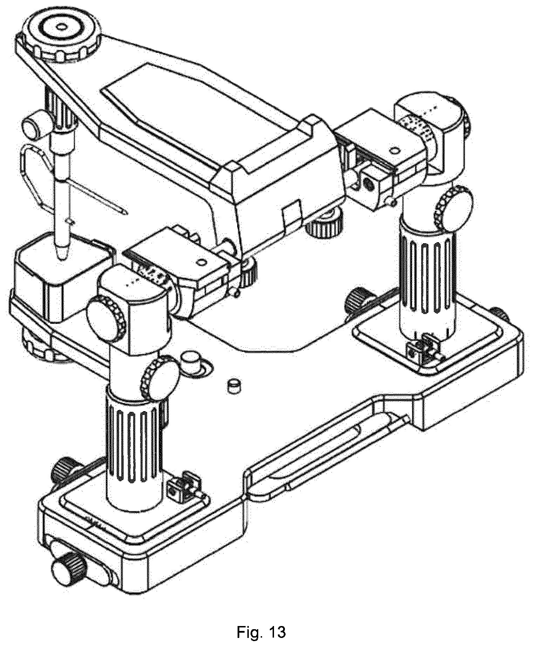

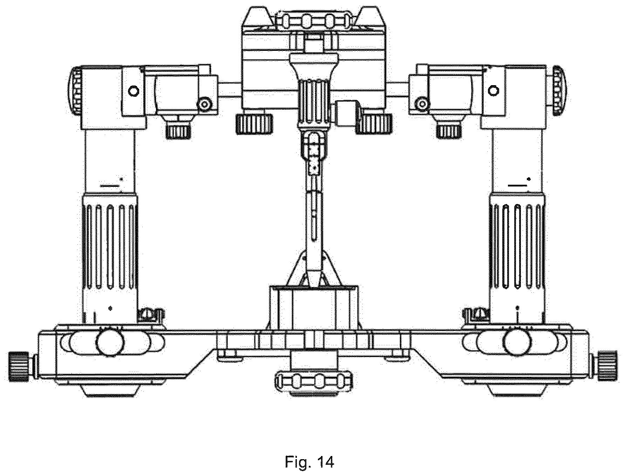

Figur 12 zeigt einen geeigneten Übertragungstisch und Fig. 13 und 14 einen Artikulator als Bewegungssimulator zur Übertragung und Simulation und einer zuvor gemessenen Relativbewegung von einem Unterkiefer (UK) relativ zu einem Oberkiefer (OK) eines Patienten.

Der Magnetfeldgenerator und die Sensorschuhe/Marker werden zum Übertragen der Positionen im Mund mittels entsprechende Software auf den Übertragungstisch genutzt, wobei der Magnetfeldgenerator zum Arbeitstisch positioniert wird. Über die Software wird die Sensor/Markerposition am Übertragungstisch mit der Position im Mund des Patienten verglichen. Es wird über die Software die korrekte Positionierung der Gipsmodelle im Übertragungstisch über die hier befestigten Sensorschuhe/Marker angezeigt.

Der Artikulator bzw. Bewegungssimulator simuliert die im Mund aufgezeichneten Bewegungen. Um bei Patienten oder auch Totalprothesenträgern eine therapeutische Position einstellen zu können, können am Bewegungssimulator ebenfalls Marker angebracht werden, die dann wieder über die Software angesteuert werden.

Über Mikrometerschrauben können x-, y-, und z- Achsen verstellt werden und so die therapeutische Situation definiert und in der Software festgehalten werden, wobei die Kondylenboxen und Kondylenachsen ebenfalls verstellbar sind, da die Kondylen bei verschiedenen Menschen unterschiedlich weit voneinander entfernt liegen.

Im Bewegungssimulator wird der Bewegungsablauf vollständig erstellt, so dass Bewegungsbahnen mit einer nach der Zahnersatzeingliederung erstellten UK-Bewegungserfassung verglichen werden.

Eine Irritation des Patienten wird vollständig vermieden und freie Bewegungen einschließlich Kauen sind erstmals bei der UK-Bewegungsaufzeichnung möglich.

### Bezugszeichenliste:

- 1: Vorrichtung
- 2: Kabelverbindung
- 3: Auswerteeinrichtung
- 4: Kopf
- 5: elektromagnetisches Messfeld
- 6: Speichereinrichtung
- 7: Schnittstelle
- 8: Senderspule
- 9: Intraoralscanner
- 10: 3D-Scanner
- D: Dreieck
- K: Koordinatensystem
- O1-8: Zähne
- OD: Dreieck
- OE: Ebene
- OK: Oberkiefer
- OS1: Oberkiefersensor
- U1-8: Zähne
- UK: Unterkiefer
- US1: Unterkiefersensor

## Patentansprüche

1. Vorrichtung (1) zur Vermessung einer Relativposition und/oder -bewegung von einem Unterkiefer (UK) relativ zu einem Oberkiefer (OK) eines Patienten, mit einer Spule (8) zum Erzeugen eines magnetischen Messfelds (5), wobei die Spule (8) extraoral seitlich oder oberhalb des Oberkiefers (OK) anordenbar ist,
mit mindestens einen Oberkiefersensor (OS1), wobei der Oberkiefersensor (OS1) intraoral auf den Zähnen (O1-O8) anordenbar ist,
mit mindestens einem Unterkiefersensor (US1), wobei der Unterkiefersensor (US1) intraoral an den Zähnen (U1-U8) anordenbar ist, wobei der Unterkiefersensor (US1) mindestens zur Bestimmung einer Position in dem Messfeld (5) relativ zu der Spule (8) ausgebildet ist, mit einer Auswerteeinrichtung (3) zur Bestimmung der Relativposition und/oder Relativbewegung des Unterkiefers (UK) relativ zu dem Oberkiefer (OK) auf Basis der von dem Unterkiefersensor (US1) bestimmten Positionen,
**gekennzeichnet durch** mehrere Halteeinrichtungen, wobei die Halteeinrichtungen am Ober- und Unterkiefer befestigbar sind und die Sensoren (OS1, US1) aufnehmen,
wobei die Halteeinrichtung an dem Oberkiefer (OK) und dem Unterkiefer (UK) als Sensorschuh ausgebildet ist, der auf die jeweiligen Zähne aufklebbar ist,
wobei der Sensorschuh zumindest einen gekrümmten Oberflächenbereich und/oder zumindest eine Positionsmarkierung aufweist, und
wobei der kleinste Abstand zwischen dem Sensor (OS1, US1) und dem jeweiligen Zahn kleiner als 0,5 cm ausgebildet ist.

2. Vorrichtung nach Anspruch 1, **dadurch gekennzeichnet, dass** die Spule (8) oberhalb oder seitlich schräg zu dem Patienten und/oder zu einem Messraum, in dem der Patient positioniert ist, anordenbar ist.

3. Vorrichtung (1) nach Anspruch 1 oder 2, **dadurch gekennzeichnet, dass** mindestens einer, einige oder alle der Sensoren (OS1, US1) jeweils zudem mindestens zwei Rotationsfreiheitsgrade, vorzugsweise alle drei Rotationsfreiheitsgrade oder sechs Freiheitsgrade in dem Messfeld (5) und/oder relativ zur Spule (8) bestimmen.

4. Vorrichtung (1) nach einem der vorhergehenden Ansprüche, **dadurch gekennzeichnet, dass** an dem Oberkiefer genau zwei fünf DOF-Sensoren oder alternativ genau ein sechs DOF-Sensor und an dem Unterkiefer genau zwei fünf DOF-Sensoren oder alternativ genau ein sechs DOF-Sensor angeordnet sind.

5. Vorrichtung (1) nach einem der vorhergehenden Ansprüche, **gekennzeichnet durch** eine Digitalisierungseinrichtung (9, 10) zur Erstellung eines digitalen, dreidimensionalen Modells des Oberkiefers (OK) und/oder des Unterkiefers (UK),
wobei in dem Modell die Halteeinrichtungen und/oder Sensoren (OS1, US1) modelliert sind,
wobei die Digitalisierungseinrichtung (9, 10) als ein Intraoralscanner (9) oder als eine Intraoralkamera ausgebildet ist.

6. Verfahren zur Bestimmung einer Relativposition und/oder -bewegung von einem Unterkiefer (UK) relativ zu einem Oberkiefer (OK) eines Patienten mit einer Vorrichtung (1) nach einem der vorhergehenden Ansprüche, umfassend die Schritte: Anordnen der Spule extraoral seitlich oder oberhalb des Oberkiefers;
Anordnen der Sensoren (OS1, US1) an dem Oberkiefer (OK) und dem Unterkiefer (UK), wobei die Halteeinrichtungen die Sensoren aufnehmen und auf die jeweiligen Zähne aufgeklebt werden;
Aufnahme von Sensorsignalen der Sensoren (OS1, US1); und Bestimmung einer Relativposition und/oder -bewegung zwischen dem Oberkiefer (OK) und dem Unterkiefer (UK) auf Basis der Sensorsignale.

7. Verfahren nach Anspruch 6, umfassend den Schritt:
Erstellung eines digitalen, dreidimensionalen Modells des Oberkiefers (OK) und/oder des Unterkiefers (UK),
wobei in dem Modell die Halteeinrichtungen und/oder Sensoren modelliert sind, sowie Fusionierung des dreidimensionalen Modells mit der bestimmten Relativposition und/oder Relativbewegung zur Erzeugung eines Abbilds des Bewegungsablaufs,
wobei der Bewegungsablauf mehrere komplexe Einzelbewegungsabläufe wie Öffnen, Schließen, Kauen etc. umfasst.

## Claims

1. Device (1) for measuring a relative position and/or relative movement of a mandible (UK) relative to a maxilla (OK) of a patient, comprising a coil (8) for generating a magnetic measurement field (5), the coil (8) being arrangeable in extraoral fashion, to the side of or above the maxilla (OK),
comprising at least one maxilla sensor (OS1), the maxilla sensor (OS1) being arrangeable in intraoral fashion on the teeth (01-08),
comprising at least one mandible sensor (US1), the mandible sensor (US1) being arrangeable in intraoral fashion on the teeth (U1-U8), the mandible sensor (ES1) being designed at least to determine a position in the measurement field (5) relative to the coil (8),
comprising an analysis unit (3) for determining the relative position and/or relative movement of the mandible (UK) relative to the maxilla (OK) on the basis of the position is determined by the mandible sensor (US1),
**characterized by** a plurality of holding units, wherein the holding units are able to be fastened to the maxilla and mandible and accommodate the sensors (OS1, US1),
wherein the holding unit on the maxilla (OK) and the mandible (UK) is designed as a sensor shoe, which is able to be adhesively bonded on the respective teeth,
wherein the sensor shoe has at least one curved surface region and/or at least one position marking, and
wherein the smallest distance between the sensor (OS1, US1) and the respective tooth is designed to be less than 0.5 cm.

2. Device according to Claim 1, **characterized in that** the coil (8) is arrangeable above or laterally diagonal in relation to the patient and/or to a measurement space, in which the patient is positioned.

3. Device (1) according to Claim 1 or 2, **characterized in that** at least one, several or all of the sensors (OS1, US1) moreover each determine at least two rotational degrees of freedom, preferably all three rotational degrees of freedom, or six degrees of freedom in the measurement field (5) and/or relative to the coil (8).

4. Device (1) according to any one of the preceding claims, **characterized in that** precisely two five DOF sensors or alternatively precisely one six DOF sensor are/is arranged on the maxilla and precisely two five DOF sensors or alternatively precisely one six DOF sensor are/is arranged on the mandible.

5. Device (1) according to anyone of the preceding claims, **characterized by** a digitizing unit (9, 10) for creating a digital, three-dimensional model of the maxilla (OK) and/or of the mandible (UK),
wherein the holding units and/or sensors (OS1, US1) are modelled on the model,
wherein the digitizing unit (9, 10) is designed as an intraoral scanner (9) or as an intraoral camera.

6. Method for determining a relative position and/or relative movement of the mandible (UK) relative to a maxilla (OK) of a patient using a device (1) according to any one of the preceding claims, comprising the steps of:
arranging the coil in extraoral fashion, to the side of or above the maxilla;
arranging the sensors (OS1, US1) on the maxilla (OK) and on the mandible (UK), wherein the holding units accommodate the sensors and are adhesively bonded on the respective teeth;
recording sensor signals of the sensors (OS1, US1); and
determining a relative position and/or relative movement between the maxilla (OK) and the mandible (UK) on the basis of the sensor signals.

7. Method according to Claim 6, comprising the step of:
creating a digital, three-dimensional model of the maxilla (OK) and/or the mandible (UK),
wherein the holding units and/or sensors are modelled on the model, and fusing the three-dimensional model with the determined relative position and/or relative movement to generate an image of the movement sequence,
wherein the movement sequence comprises multiple complex individual movement sequences such as opening, closing, chewing, etc.

## Revendications

1. Arrangement (1) pour mesurer une position relative et/ou un mouvement relatif d'une mâchoire inférieure (UK) par rapport à une mâchoire supérieure (OK) d'un patient, comprenant une bobine (8) destinée à générer un champ de mesure (5) magnétique, la bobine (8) pouvant être disposée de manière extraorale latéralement ou au-dessus de la mâchoire supérieure (OK),
comprenant au moins un capteur de mâchoire supérieure (OS1), le capteur de mâchoire supérieure (OS1) pouvant être disposé de manière intraorale sur les dents (01-08), comprenant au moins un capteur de mâchoire inférieure (US1), le capteur de mâchoire inférieure (US1) pouvant être disposé de manière intraorale sur les dents (U1-U8), le capteur de mâchoire inférieure (US1) étant configuré au moins pour la détermination d'une position dans le champ de mesure (5) par rapport à la bobine (8), comprenant un dispositif d'interprétation (3) destiné à déterminer la position relative et/ou le mouvement relatif de la mâchoire inférieure (UK) par rapport à la mâchoire supérieure (OK) sur la base des positions déterminées par le capteur de mâchoire inférieure (US1), **caractérisé par** plusieurs dispositifs de maintien, les dispositifs de maintien pouvant être fixés à la mâchoire supérieure et inférieure et accueillant les capteurs (OS1, US1),
le dispositif de maintien au niveau de la mâchoire supérieure (OK) et de la mâchoire inférieure (UK) étant réalisé sous la forme d'un sabot de capteur qui peut être collé sur les dents respectives,
le sabot de capteur possédant au moins une zone de surface courbe et/ou au moins un repérage de position et
le plus petit écart entre le capteur (OS1, US1) et la dent respective étant formé inférieur à 0,5 cm.

2. Arrangement selon la revendication 1, **caractérisé en ce que** la bobine (8) peut être disposée au-dessus ou latéralement en biais par rapport au patient et/ou par rapport à un espace de mesure dans lequel est positionné le patient.

3. Arrangement (1) selon la revendication 1 ou 2, **caractérisé en ce qu'**au moins un, certains ou tous les capteurs (OS1, US1) déterminent respectivement en plus au moins deux degrés de liberté de rotation, de préférence tous les trois degrés de liberté de rotation ou six degrés de liberté dans le champ de mesure (5) et/ou par rapport à la bobine (8).

4. Arrangement (1) selon l'une des revendications précédentes, **caractérisé en ce qu'**au niveau de la mâchoire supérieure sont disposés exactement deux capteurs à cinq degrés de liberté ou, en variante, exactement un capteur à six degrés de liberté, et au niveau de la mâchoire inférieure exactement deux capteurs à cinq degrés de liberté ou, en variante, exactement un capteur à six degrés de liberté.

5. Arrangement (1) selon l'une des revendications précédentes, **caractérisé par** un dispositif de numérisation (9, 10) destiné à créer un modèle numérique tridimensionnel de la mâchoire supérieure (OK) et/ou de la mâchoire inférieure (UK),
les dispositifs de maintien et/ou les capteurs (OS1, US1) étant modélisés dans le modèle,
le dispositif de numérisation (9, 10) étant réalisé sous la forme d'un appareil de numérisation intraoral (9) ou sous la forme d'une caméra intraorale.

6. Procédé de détermination d'une position relative et/ou d'un mouvement relatif d'une mâchoire inférieure (UK) par rapport à une mâchoire supérieure (OK) d'un patient avec un arrangement (1) selon l'une des revendications précédentes, comprenant les étapes suivantes :
disposition des capteurs (OS1, US1) au niveau de la mâchoire supérieure (OK) et de la mâchoire inférieure (UK), les dispositifs de maintien accueillant les capteurs et étant collés sur les dents respectives ;
enregistrement de signaux de capteur des capteurs (OS1, US1) ; et
détermination d'une position relative et/ou d'un mouvement relatif entre la mâchoire supérieure (OK) et la mâchoire inférieure (UK) sur la base des signaux de capteur.

7. Procédé selon la revendication 6, comprenant l'étape suivante :
création d'un modèle numérique tridimensionnel de la mâchoire supérieure (OK) et/ou de la mâchoire inférieure (UK),
les dispositifs de maintien et/ou les capteurs étant modélisés dans le modèle, ainsi que fusion du modèle tridimensionnel avec la position relative et/ou le mouvement relatif déterminé en vue de générer une représentation du déroulement du mouvement,
le déroulement du mouvement comportant plusieurs déroulements de mouvement complexes, comme l'ouverture, la fermeture, la mastication, etc.
